# EUROPEAN PATENT SPECIFICATION

(11) **EP 4 554 529 B1**
(45) Date of publication and mention of the grant of the patent: **01.04.2026**
(21) Application number: 23744671.1
(22) Date of filing: 13.07.2023
(51) Int. Cl.: A61F 5/445

(54) **SENSOR PATCH FOR AN OSTOMY APPLIANCE HAVING AUXETIC CUTS**
SENSORPFLASTER FÜR EINE STOMAVORRICHTUNG MIT AUXETISCHEN SCHNITTEN
PATCH CAPTEUR POUR UN APPAREIL DE STOMIE AYANT DES COUPES AUXÉTIQUES

(30) Priority: 14.07.2022 DK PA202270378
(43) Date of publication of application: 21.05.2025
(73) Proprietor: Coloplast A/S, 3050 Humlebaek (DK)
(72) Inventor: STROEBECH, Esben, 2970 Hoersholm (DK); SUND, Anders Grove, 3450 Alleroed (DK)
(86) International application number: PCT/DK2023/050186
(87) International publication number: WO 2024/012644

(56) References cited:
- WO-A1-2020/156624
- WO-A1-2020/156625
- WO-A1-2020/156626

## Description

### TECHNICAL FIELD

The present disclosure relates to a sensor patch for an ostomy appliance and a method of manufacturing such a sensor patch.

### BACKGROUND

Stomal output often contains body fluids and visceral contents that are aggressive to both the skin of a user and to ostomy devices. These have a detrimental effect on the efficiency and integrity of the adhesive materials that are applied to attach the ostomy device to the user's skin surface. For users in general, safe, reliable and efficient ostomy devices are evidently highly desirable.

However, a particularly major and persistent concern of a large population of ostomists continues to be failure of the base plate adhesive attaching the ostomy appliance to the user's skin surface, because such failure almost inevitably leads to embarrassing and stigmatising leakage incidents. Such incidents in turn are known from several user interviews to lead to a reduced quality-of-life feeling. Adhesive failure of the base plate adhesive can result from various reasons. Most often, a leakage incident is caused by stomal output entering between the proximal surface of the base plate and the user's skin, e.g. due to less-than-optimal attachment of the base plate to the skin arising from e.g. uneven skin surface or skin folds. This undesirable progression of stomal output "underneath" the adhesive leads to deterioration and/or weakening of the adhesive material carrying the weight and providing the seal of the ostomy appliance. Often, such failure happens surprisingly fast and is only detectable for the user once the failure has already become so severe that leakage occurs, requiring immediate change of the ostomy appliance and possibly also of the user's clothes. In other instances, the primary factor of adhesive failure is simply a question of how much time has elapsed since the base plate of the ostomy appliance was first applied to the user's skin surface. In addition to the output from the stoma itself, the peristomal skin surface continuously secretes some moisture (e.g. sweat). To mitigate this, most often adhesives of base plates for ostomy devices include hydrocolloid materials which are capable of absorbing high levels of moisture, thereby stabilizing the polymer matrix of the adhesive material and prolonging the lifetime ("wear time") of the base plate. However, eventually the adhesion capability of the base plate no longer can support the force exerted on the base plate from the load of the output collecting bag, and the appliance must be replaced.

WO 2020/156626 discloses a sensor patch for attaching to a base plate of an ostomy appliance, which has a planar adhesive layer that is provided with a proximal side and a distal side with main spatial shape, and electrodes are arranged on the distal side of the adhesive layer.

As there can be considerable differences in the severity and/or speed by which adhesive failure and potentially leakage occur, which differences at least to some extent are correlated to various factors including those presented above, a mere indication that failure or leakage is imminent, or that it has already occurred, fails to represent a reliable and satisfactory solution to the problem of avoiding sudden embarrassing and stigmatising leakage incidents in ostomy appliances. In other words, the users of ostomy appliances could greatly benefit from an appliance solution which provides them with better guidance and options regarding how and - not least - how quickly to react to beginning failure or leakage of the adhesive of the base plate of the appliance. More generally, ostomists and health care professionals alike would welcome improvements in ostomy devices to reduce or eliminate the occurrence of sudden leakage incidents.

### SUMMARY

On this background, it may be seen as an object of the present disclosure to provide a sensor patch for facilitating reliable and/or improved detection of risk of failure of an ostomy appliance and/or improved detection of risk of leakage, especially when the stoma of the user requires the use of a convex or concave base plate adapted to fit the shape/contours of the peristomal area. Another object of the present disclosure is to provide a method of manufacturing such a sensor patch.

One or more of these objects may be met by aspects of the present disclosure as described in the following.

A first aspect of this disclosure relates to a sensor patch for attachment to a concave or convex base plate for an ostomy appliance, the sensor patch having a proximal side and a distal side, the distal side being adapted for attachment to an adhesive surface of the base plate, wherein the adhesive surface of the base plate is adapted for attachment of the base plate to the skin surface of a user, wherein the sensor patch is adapted to form a stomal opening with a centre point, the stomal opening being configured to allow passage of output through the stomal opening and into an ostomy pouch attached to the base plate, the sensor patch comprising:
- a support layer with a distal surface and a proximal surface;
- a plurality of electrode paths for forming one or more sensors, the plurality of electrode paths being arranged on the proximal surface of the support layer; and
- an adhesive layer arranged on the proximal surface of the support layer;
wherein the support layer is auxetic so as to allow conforming the sensor patch to the concave or convex base plate.

An auxetic support layer may improve the conformability of the sensor patch to three-dimensionally contoured surfaces with reduced creasing. This is particularly advantageous when the base plate, to which the sensor patch is fitted, is convex or concave.

In the context of the present disclosure, "auxetic" may refer to a macroscopic structure or material which has a negative Poisson's ratio. When stretched, such structures or materials become thicker perpendicular to the applied force. This occurs due to their particular internal structure and the way this deforms when the sample is uniaxially loaded.

Additionally, the auxetic property of the support layer may be provided by a plurality of openings extending through the support layer and being spaced apart from each other.

Providing the support layer with a plurality of openings is a cost-effective approach to achieving the auxetic property of the support layer.

Additionally, the plurality of openings may also extend through the adhesive layer. Thus, in embodiments, the adhesive layer is auxetic/has an auxetic property, such as in addition to the support layer. This may enhance the benefits as disclosed herein in relation to the auxetic property of the support layer.

Alternatively, the auxetic property of the support layer may be provided by including folds in the material of the support layer or including an auxetic network structure in the material of the support layer.

Additionally or alternatively, each opening of the plurality of openings may comprise at least a first opening portion and a second opening portion both extending from a common start point to a respective end point. The end point of the first opening portion may be spaced from the end point of the second opening portion. The common start point of a first opening of the plurality of openings may be arranged on a first side of an imaginary straight line extending in a direction on the support layer and the common start point of a second opening of the plurality of openings may be arranged on a second side of the straight line. The first opening portion of the first opening and the second opening portion of the second opening may each cross the straight line.

It has been found that providing the openings in this way, provides the support layer with excellent conformability.

Additionally or alternatively, the plurality of openings may form a lattice pattern. The lattice pattern may preferably be translational symmetric.

In other words, each opening of the plurality of openings has the same dimensions and shape as the other openings provided in the support layer.

Additionally or alternatively, the plurality of electrode paths may extend circumferentially at least partially, but preferably substantially entirely, e.g. 90% or more, around the stomal opening.

Additionally or alternatively, at least one of the electrode paths may extend between the first opening and the second opening and may preferably cross the straight line.

Providing the electrode paths between the openings allows for arranging the electrodes in close contact with the concave or convex base plate to provide an improved sensor signal without requiring a restriction of the material of the electrode paths to only highly stretchable or conformable materials.

Additionally or alternatively, each opening portion of the plurality of openings may be formed as a slit.

Additionally or alternatively, an angle between one of the opening portions of an opening and another opening portion of the same opening may be between 60° and 150°.

Additionally or alternatively, each of the plurality of openings may further comprise a third opening portion extending from the common start point to a respective end point. Additionally, an angle between the second opening portion and the third opening portion of the same opening may be between 60° and 120°.

Additionally or alternatively, each of the plurality of openings may be substantially shaped as a star, preferably a three-pointed star.

Additionally or alternatively, the sensor patch may further comprise a release liner arranged on the proximal surface of the adhesive layer. In the case that the plurality of openings extends through the adhesive layer, the release liner may comprise a plurality of protrusions each extending into a respective opening of the plurality of openings. Such protrusions may support the plurality of openings, e.g. during storage, since the adhesive material of the adhesive layer may be liquid to some degree.

Additionally or alternatively, the support layer may be moisture permeable. Alternatively, the support layer may be moisture non-permeable.

Additionally or alternatively, the support layer is a non-woven material and/or a perforated polymer-film material.

Additionally or alternatively, the support layer may be made of a polymer film, preferably polyurethane film.

A second aspect of this disclosure relates to a method for manufacturing a sensor patch according to the first aspect of this disclosure. The method comprising the steps of:
- providing a support layer with a distal surface and a proximal surface; and
- arranging a plurality of electrode paths on the proximal surface of the support layer, the plurality of electrode paths may extend circumferentially around the stomal opening.

Additionally, the support layer may be provided with the plurality of openings being pre-made.

Additionally or alternatively, the method may comprise a step of cutting the support layer to provide the plurality of openings in the support layer.

Additionally or alternatively, the step of arranging the plurality of electrode paths on the proximal surface of the support layer may comprise arranging the plurality of electrode paths to extend between the plurality of openings.

A third aspect of the present disclosure relates to a kit of parts for an ostomy appliance, the kit of parts comprising:
- a sensor patch according to the first aspect of this disclosure; and
- a concave or convex base plate comprising an adhesive surface adapted for attachment to a skin surface of a user, the base plate forming a stomal opening with a centre point, the stomal opening being configured to allow passage of output from the user through the stomal opening and into an ostomy pouch attached to the base plate.

Additionally, the distal surface of the sensor patch may be attached to the convex or concave adhesive surface of the base plate substantially without creases.

A person skilled in the art will appreciate that any one or more of the above aspects of this disclosure and embodiments thereof may be combined with any one or more of the other aspects of this disclosure and embodiments thereof.

### BRIEF DESCRIPTION OF THE DRAWINGS

Embodiments of this disclosure will be described in more detail in the following with regard to the accompanying figures. The figures show one way of implementing the present invention and are not to be construed as being limiting to other possible embodiments falling within the scope of the attached claim set.
Fig. 1 schematically illustrates an exploded view of an exemplary two-piece ostomy appliance.
Fig. 2 schematically illustrates an exploded view of an exemplary two-piece ostomy appliance comprising a sensor patch.
Fig. 3 schematically illustrates an exemplary electrode path configuration.
Fig. 4 schematically illustrates a kit of parts including a convex base plate and an exemplary sensor patch comprising the support layer with an exemplary plurality of openings.
Fig. 5A schematically illustrates a section of an exemplary lattice pattern of the exemplary plurality of openings of the sensor patch shown in Fig. 4.
Fig. 5B schematically illustrates two adjacent openings of the plurality of openings shown in Fig. 5A.

### DETAILED DESCRIPTION OF THE INVENTION

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used with respect to the orientation of the Figure(s) being described. Because components of embodiments can be positioned in a number of different orientations, the directional terminology is used for purposes of illustration and is in no way limiting. It is to be understood that other embodiments may be utilized, and structural or logical changes may be made without departing from the scope of the present invention. The following detailed description, therefore, is not to be taken in a limiting sense, and the scope of the present invention is defined by the appended claims.

It is to be understood that the features of the various exemplary embodiments described herein may be combined with each other, unless specifically noted otherwise.

Throughout this disclosure, the words "stoma" and "ostomy" are used to denote a surgically created opening bypassing the intestines or urinary tract system of a person. The words are used interchangeably, and no differentiated meaning is intended. The same applies for any words or phrases derived from these, e.g. "stomal", "ostomies" etc. Also, the solid and liquid wastes emanating from the stoma may be referred to as both stomal "output", "waste(s)", and "fluids" interchangeably. A subject having undergone ostomy surgery may be referred to as "ostomist" or "ostomate" - moreover, also as "patient" or "user". However, in some cases "user" may also relate or refer to a health care professional (HCP), such as a surgeon or an ostomy care nurse or others. In those cases, it will either be explicitly stated or be implicit from the context that the "user" is not the "patient" him- or herself.

In the following, whenever referring to proximal side of a device or part of a device, the referral is to the skin-facing side, when the ostomy appliance is worn by a user. Likewise, whenever referring to the distal side of a device or part of a device, the referral is to the side facing away from the skin, when the ostomy appliance is worn by a user. In other words, the proximal side is the side closest to the user when the appliance is fitted on a user, and the distal side is the opposite side - the side furthest away from the user when the appliance is fitted on the user.

The axial direction is defined as the direction of the stoma when the appliance is worn by a user. Thus, the axial direction is generally perpendicular to the skin or abdominal surface of the user.

The radial direction is defined as transverse to the axial direction that is transversely to the direction of the stoma, i.e. "across" the distal/proximal surface of the base plate. In some sentences, the words "inner" and "outer" may be used. These qualifiers should generally be perceived with respect to the radial direction, such that a reference to an "outer" element means that the element is farther away from a centre portion of the ostomy appliance than an element referenced as "inner". In addition, "innermost" should be interpreted as the portion of a component forming a centre of the component and/or being adjacent to the centre of the component. In analogy, "outermost" should be interpreted as a portion of a component forming an outer edge or outer contour of a component and/or being adjacent to that outer edge or outer contour.

The use of the word "substantially" as a qualifier to certain features or effects in this disclosure is intended to simply mean that any deviations are within tolerances that would normally be expected by the skilled person in the relevant field.

The use of the word "generally" as a qualifier to certain features or effects in this disclosure is intended to simply mean - for a structural feature: that a majority or major portion of such feature exhibits the characteristic in question, and - for a functional feature or an effect: that a majority of outcomes involving the characteristic provide the effect, but that exceptionally outcomes do not provide the effect.

The use of the word "essentially" as a qualifier to certain structural and functional features or effects in this disclosure is used for emphasizing what is the most important focus of something or fact about something (i.e. a feature may have or fulfil a variety of effects, but when the disclosure discusses one effect as being "essentially" provided, this is the focus and the most important effect in relation to the disclosure).

Throughout the disclosure, the use of the terms "first", "second", "third", "fourth", "primary", "secondary", "tertiary" etc. does not imply any particular order or importance but is included merely to identify individual elements. Furthermore, the labelling of a first element does not imply the presence of a second element and vice versa.

Disclosed is an ostomy appliance which can either be a one-piece ostomy appliance or a two-piece ostomy appliance. The ostomy appliance comprises a base plate and an ostomy pouch (also referred to as an ostomy bag). The base plate is configured for coupling to a user's stoma and/or skin surrounding the stoma, such as a peristomal skin area. The ostomy appliance may be a colostomy appliance, an ileostomy appliance or a urostomy appliance. The ostomy appliance may be a two-piece ostomy appliance, i.e. the base plate and the ostomy pouch may be releasably coupled e.g. with a mechanical and/or an adhesive coupling, e.g. to allow that a plurality of ostomy pouches can be utilized (exchanged) with one base plate. For example, the base plate may comprise a coupling ring for coupling an ostomy pouch to the base plate. Further, a two-piece ostomy appliance may facilitate correct application of the base plate to skin, e.g. to an improved user sight of the stomal region. Alternatively, the ostomy appliance may be a one-piece ostomy appliance, i.e. the base plate and the ostomy pouch may be fixedly attached to each other. The one-piece or two-piece ostomy appliance may further include a separate sensor patch for attachment to the base plate. The sensor patch may facilitate detection of moisture propagation in an adhesive material as well as detection of a heightened risk of leakage. For example, the sensor patch may allow electronic measurements of performance of the base plate and/or to facilitate detection of increasing risks of leakage and/or to facilitate detection of decreasing adherence of the base plate to the skin of the user.

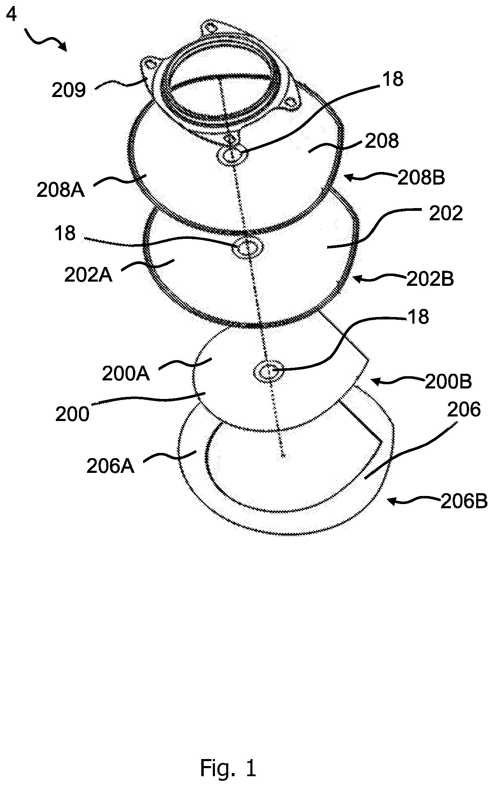

Fig. 1 schematically illustrates an exploded view of an exemplary two-piece ostomy appliance comprising a base plate 4. The base plate 4 comprises a first adhesive layer 200 having a distal surface 200A and a proximal surface 200B. During use, the proximal surface 200B of the first adhesive layer 200 adheres to the user's skin. The base plate 4 comprises a second adhesive layer 202 having a distal surface 202A and a proximal surface 202B. As illustrated, the second adhesive layer 202 spans a larger surface area than the first adhesive layer 200, such as to provide a rim of the proximal surface 202B of the second adhesive layer 202 surrounding the proximal surface 200B of the first adhesive layer 200.

The base plate 4 comprises a release liner 206, which may be peeled off by the user prior to applying the base plate 4 to the skin. The release liner 206 comprises a distal surface 206A and a proximal surface 206B. The distal surface 206A of the release liner 206 is covering the proximal surface of the first adhesive layer 200 and covering the proximal surface of the second adhesive layer 202 not covered by the first adhesive layer 200.

The base plate 4 comprises a backing layer 208. The backing layer 208 is a protective layer protecting the adhesive layers, such as the first adhesive layer 200 and/or the second adhesive layer 202 from external strains and stress during use. Furthermore, the backing layer 208 also covers the adhesive layers, such as the first adhesive layer 200 and/or the second adhesive layer 202, such that the adhesive layers 200, 202 do not adhere to clothes worn on top of the base plate 4. The backing layer 208 comprises a distal surface 208A and a proximal surface 208B. The distal surface 208A of the backing layer 208 is configured to face away from the skin of the user. The proximal surface 208B of the backing layer 208 is covering the second adhesive layer 202.

The base plate 4 forms part of a two-piece ostomy appliance, thus comprising a coupling ring 209 for coupling an ostomy pouch to the base plate 4, such as to a distal side of the base plate 4.

The base plate 4 comprises a stomal opening. The layers of the base plate 4, such as the first adhesive layer 200, the second adhesive layer 202 and the backing layer 208 as illustrated, may comprise stomal openings 18 for collectively forming the stomal opening of the base plate.

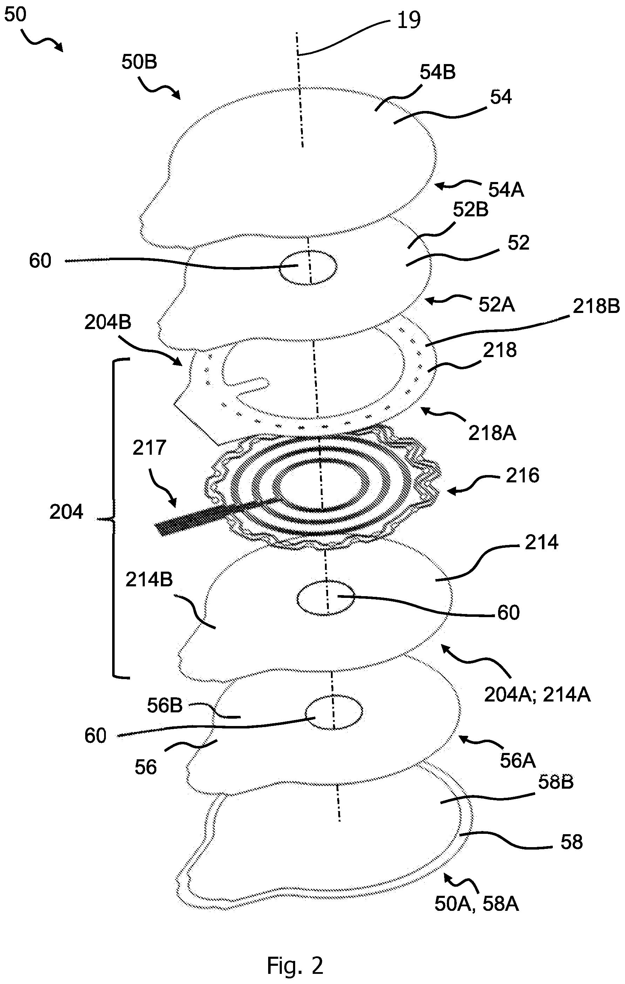

Fig. 2 schematically illustrates an exploded view of an exemplary two-piece ostomy appliance comprising a sensor patch 50, such as a sensor patch 50 being adapted for attachment to a base plate, such as the base plate 4 as illustrated in Fig. 1. The sensor patch 50 is configured to be positioned between the skin of the user and the proximal side of the base plate 4. For example, the sensor patch may be adapted for attachment to the first adhesive layer 200, such as the proximal surface 200B of the first adhesive layer 200, of the base plate 4 as illustrated in Fig. 1. The sensor patch 50 is configured to be attached to the base plate such that the distal side 50A of the sensor patch 50 is attached to the proximal side of the base plate, such as to the proximal surface 200B of the first adhesive layer 200 of the base plate 4.

The sensor patch 50 comprises a sensor assembly 204 comprising a plurality of electrode paths 216. Each electrode path 216 has respective connection parts 217 for connecting the plurality of electrode paths 216 to respective terminal elements of a monitor device. The sensor assembly 204 may form a sensor assembly layer.

The sensor assembly 204 has a distal side 204A and a proximal side 204B. The sensor assembly 204 comprises a support layer 214 with a proximal surface 214B. The electrode paths 216 may be provided, such as formed, on the proximal surface 214B of the support layer 214, e.g. the electrode paths 216 may be positioned on the proximal surface 214B of the support layer 214.

The electrode path assembly 204 comprises a masking element 218 having a distal surface 218A and a proximal surface 218B. The masking element 218 is configured to electrically insulate at least parts of the electrode paths 216 from adjacent layers, such as the first adhesive sensor layer 52. The masking element 218 covers or overlaps with parts of the electrode paths 216 when seen in the axial direction.

The sensor patch 50 comprises a first adhesive sensor layer 52, with a proximal side 52B and a distal side 52A. The first adhesive sensor layer 52 is arranged on the proximal side 204B of the sensor assembly 204. The proximal side 52B of the first adhesive sensor layer 52 is configured to adhere to the user's skin. Thus, after being applied to the base plate, the combined base plate and sensor patch 50 form an adhesive proximal surface configured to be applied to the skin surface of the user.

The sensor patch comprises a first sensor release liner 54. The first sensor release liner 54 may comprise a distal surface 54A and a proximal surface 54B. The first sensor release liner 54 may be arranged to protect the first adhesive sensor layer 52. The distal surface 54A of the first sensor release liner 54 is facing the proximal surface 52B of the first adhesive sensor layer 52. The first sensor release liner 54 is configured to be peeled off by the user prior to application of the base plate with the attached sensor patch to the skin. The first adhesive sensor layer 52 may be laid out on the distal side 54A of the first sensor release liner 54.

The sensor patch 50 comprises a second adhesive sensor layer 56, with a proximal side 56B and a distal side 56A. The second adhesive sensor layer 56 is arranged on the distal side 204A of the sensor assembly 204. The proximal side 56B of the second adhesive sensor layer 52 is configured to adhere to the base plate, such as the proximal surface of the first adhesive layer of the base plate.

The exemplary sensor patch comprises a second sensor release liner 58. The second sensor release liner 58 may comprise a distal surface 58A and a proximal surface 58B. The second sensor release liner 58 may be arranged to protect the second adhesive sensor layer 56. The proximal surface 58B of the second sensor release liner 58 is facing the distal surface 56A of the second adhesive sensor layer 56. The second sensor release liner 58 is configured to be peeled off by the user prior to application of the sensor patch to the base plate. The second adhesive sensor layer 56 may be laid out on the proximal side 58B of the second sensor release liner 58.

Whereas the illustrated exemplary sensor patch 50 comprises a second adhesive sensor layer 56 arranged on the distal side 204A of the sensor assembly 204, it is envisioned that an alternative sensor patch according to the disclosure may be provided without such a second adhesive sensor layer 56 and an accompanying second sensor release liner 58. Rather, in such an alternative sensor patch, it is envisioned that the distal surface of the sensor patch is the distal surface of the sensor assembly 204, in particular the distal surface of the support layer 214. In such an alternative sensor patch, the distal surface of the support layer 214 may be configured for attachment to the adhesive surface of a base plate. Thus, whereas a sensor patch 50 having a first 52 and a second adhesive sensor layer 56 is illustrated and described, it is envisioned that a similar sensor patch without the second adhesive sensor layer 56 and accompanying second sensor release liner 58 may be provided according to the disclosure.

The sensor patch 50 comprises a stomal opening. The layers of the sensor patch 50, such as the first adhesive sensor layer 52, the support layer 214 and the second adhesive sensor layer 56, as illustrated, may comprise stomal openings 60 for collectively forming the stomal opening of the sensor patch 50. The stomal opening of the sensor patch is configured to be aligned with the stomal opening 18 of the base plate, such as to collectively form the stomal opening of the combined base plate and sensor patch 50.

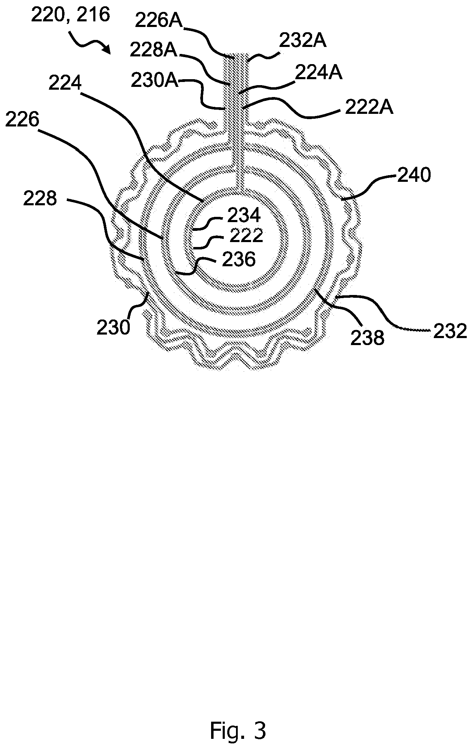

Fig. 3 schematically illustrates an exemplary electrode path configuration 220 of electrode paths 216 of an exemplary sensor assembly, such as the sensor assembly 204 as described with respect to Fig. 2. The plurality of electrode paths 216 comprises a first electrode path 222, a second electrode path 224, a third electrode path 226, a fourth electrode path 228, a fifth electrode path 230, and a sixth electrode path 232.

The first electrode path 222 comprises a first connection part 222A and the second electrode path 224 comprises a second connection part 224A. The third electrode path 226 comprises a third connection part 226A. The fourth electrode path 228 comprises a fourth connection part 228A. The fifth electrode path 230 comprises a fifth connection part 230A. The sixth electrode path 232 comprises a sixth connection part 232A.

The first electrode path 222 may be a common ground electrode, also denoted a reference electrode, such as to form sensors with respect to the remaining electrode paths. The first electrode path 222 comprises a first electrode part 234 for forming a ground for the second electrode path 224. The first electrode path 222 comprises a second electrode part 236 for forming a ground for the third electrode path 226. The first electrode path 222 comprises a third electrode part 238 for forming a ground for the fourth electrode path 228. The first electrode path 222 comprises a fourth electrode part 240 for forming a ground for the fifth electrode path 230 and the sixth electrode path 232.

As shown in Fig. 2, the sensor patch 50 comprises a stomal opening 60 with a centre point 19 (here illustrated as by a centre line). In some exemplary sensor patches, the stomal opening 60 may be made by the user, i.e. the sensor patch may be manufactured and/or sold without the stomal opening 60, but with a region adapted to form the stomal opening 60 with the centre point 19.

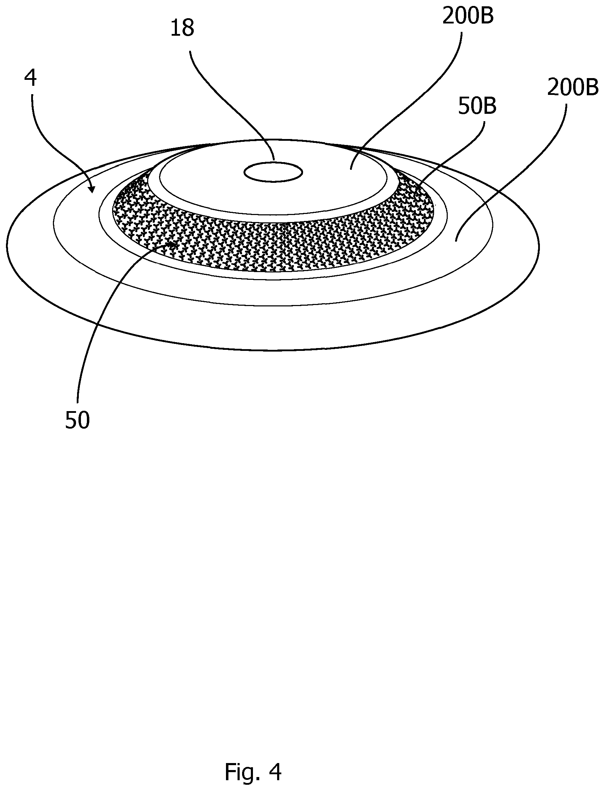

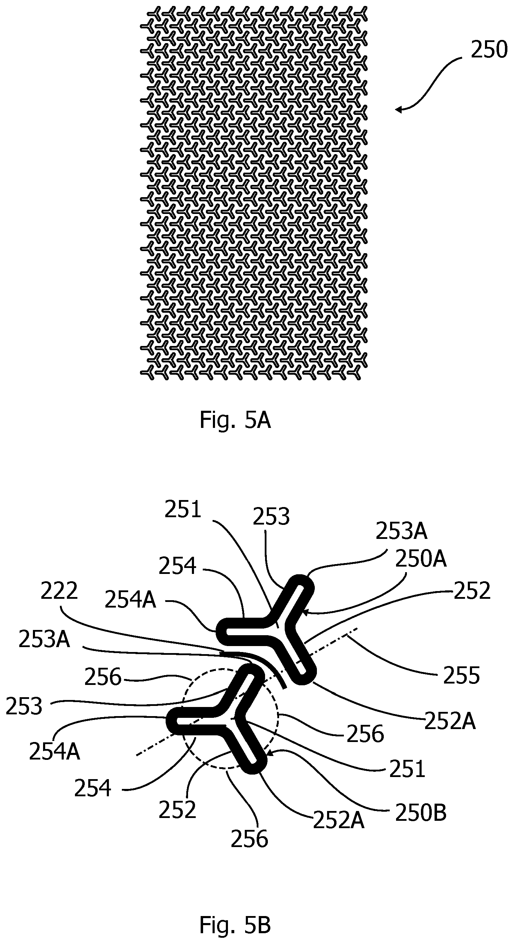

Turning to Fig. 4, a kit of parts for an ostomy appliance is shown. The kit of parts comprises a convex base plate 4 and a sensor patch 50. As described above, the base plate 4 comprises a backing layer 208, a first adhesive layer 200, and a second adhesive layer 202. The sensor patch 50 comprises a support layer 214, a plurality of electrode paths (not shown), and an adhesive layer (not shown). The support layer 214 is auxetic so as to allow conforming the sensor patch 50 to the convex base plate 4 (i.e. adapted for a user with a convex or inwardly stoma). In other embodiments, the same auxetic sensor patch 50 can conform to a concave base plate 4 (i.e. adapted for a user with a concave or outwardly stoma). Since a convex or concave base plate 4 has a contoured three-dimensionally shaped proximal surface, a conventional sensor patch cannot easily conform to this surface without creasing. However, an auxetic sensor patch 50 as shown in Fig. 4, can easily conform without any substantial creasing. The auxetic property of the support layer 214 is in the present embodiment provided by a plurality of openings 250, as shown in Fig. 5A, being spaced apart and extending through the support layer 214 and optionally also the adhesive layer. In other embodiments, the auxetic property may be provided by including folds in the material of the support layer 214 or including an auxetic molecular network structure in the material of the support layer 214.

Since Fig. 4 is shown in a perspective view, the arrangement of the plurality of openings are slightly distorted in this view. The arrangement of the plurality of openings 250 is more clearly seen from the top-down view of Fig. 5A. As seen, the plurality of openings 250 is arranged in a translational symmetric lattice pattern. In other words, each opening of the plurality of openings has the same dimensions and shape as the other openings provided in the support layer. The relative arrangement of two adjacent openings of this lattice pattern is best seen in Fig. 5B showing a first and second adjacent opening 250A, 250B. This relative arrangement applies for each opening pair of the lattice pattern. As seen, each opening 250A, 250B of the plurality of openings 250 comprises a first opening portion 252, a second opening portion 253, and a third opening portion 254 all extending from a common start point 251 (e.g. the centre point of each opening) to a respective end point 252A, 253A, 254A. Each opening portion can be seen as a slit started from the common start point 251. All end points 252A, 253A, 254A are spaced apart from each other, for example the end point 252A of the first opening portion 252 is spaced from the end points 253A, 254A of the second opening portion 253 and third opening portion 254. In the present embodiment, the angle 256 between each of the opening portions is 120° and each opening 250A, 250B may resemble a three-pointed star. However, the openings may have various other shapes in other embodiments.

The relative arrangement of adjacent openings is as follows. An imaginary straight line 255 is conceptually drawn extending in a direction on the support layer 214 between the common start points 251 of the first and second opening 250A, 250B. Accordingly, the common start point 251 of the first opening 250A is arranged on a first side of the imaginary straight line 255 and the common start point 251 of the second opening 250B is arranged on a second side (i.e. on the opposite side) of the imaginary straight line 255. The opening portions of the openings are arranged so that the first opening portion 252 of the first opening 250A and the second opening portion 253 of the second opening 250B each cross the straight line 255. In other words, the common start point 251 of the first opening 250A and the end point 252A of the first opening portion 252 of the first opening 250A lies on opposite sides of the imaginary straight line 255. Similarly, the common start point 251 of the second opening 250B and the end point 253B of the second opening portion 253 of the second opening 250B lies on opposite sides of the imaginary straight line 255. In the shown embodiment, the third opening portion 254 of the second opening 250B also crosses the straight line 255 (i.e. the common start point 251 of the second opening 250B and the end point 254A of the third opening portion 254 of the second opening 250B lies on opposite sides of the imaginary straight line 255).

Fig. 5B further illustrates a section of an electrode path 222 extending between adjacent openings 250A, 250B and crossing the imaginary straight line 255 to allow the provision of one or more sensors of the sensor patch 50. A plurality of electrode paths can thus extend in a similar manner in the lattice pattern of the plurality of openings 250 of Figs. 4 and 5A to form an electrode configuration 220 similar the one shown in Fig. 3 wherein the concentric circles of the electrode paths may instead zigzag between the plurality of openings to form substantially concentric circles.
- 4: base plate
- 18: stomal opening
- 19: centre point
- 50: sensor patch
- 50A: distal side
- 50B: proximal side
- 52: first adhesive sensor layer
- 52B: proximal side
- 52A: distal side
- 54: first sensor release liner
- 54A: distal surface
- 54B: proximal surface
- 56: second adhesive sensor layer
- 56B: proximal side
- 56A: distal side
- 58: second sensor release liner
- 58A: distal surface
- 58B: proximal surface
- 60: stomal opening
- 200: first adhesive layer
- 200A: distal surface
- 200B: proximal surface
- 202: second adhesive layer
- 202A: distal surface
- 202B: proximal surface
- 204: sensor assembly
- 204A: distal side
- 204B: proximal side
- 206: release liner
- 206A: distal surface
- 206B: proximal surface
- 208: backing layer
- 208A: distal surface
- 208B: proximal surface
- 209: coupling ring
- 214: support layer
- 214A: distal surface
- 214B: proximal surface
- 216: electrode path
- 217: connection part
- 218: masking element
- 218A: distal surface
- 218B: proximal surface
- 220: electrode path configuration
- 222: first electrode path
- 222A: first connection part
- 224: second electrode path
- 224A: second connection part
- 226: third electrode path
- 226A: third connection part
- 228: fourth electrode path
- 228A: fourth connection part
- 230: fifth electrode path
- 230A: fifth connection part
- 232: sixth electrode path
- 232A: sixth connection part
- 234: first electrode part
- 236: second electrode part
- 238: third electrode part
- 240: fourth electrode part
- 250: plurality of openings
- 250A: first opening
- 250B: second opening
- 251: common start point
- 252: first opening portion
- 252A: end point
- 253: second opening portion
- 253A: end point
- 254: third opening portion
- 254A: end point
- 255: imaginary straight line
- 256: opening portion angle

## Claims

1. A sensor patch (50) for attachment to a concave or convex base plate for an ostomy appliance,
the sensor patch having a proximal side and a distal side, the distal side being adapted for attachment to an adhesive surface of the base plate, wherein the adhesive surface of the base plate is adapted for attachment of the base plate to the skin surface of a user, wherein the sensor patch is adapted to form a stomal opening (18) with a centre point, the stomal opening being configured to allow passage of output through the stomal opening and into an ostomy pouch attached to the base plate, the sensor patch comprising:
- a support layer (214) with a distal surface (214A) and a proximal surface (214B);
- a plurality of electrode paths (216) for forming one or more sensors, the plurality of electrode paths being arranged on the proximal surface of the support layer; and
- an adhesive layer (52, 56) arranged on the proximal surface of the support layer;
**characterised in that**
the support layer is auxetic so as to allow conforming the sensor patch to the concave or convex base plate.

2. The sensor patch according to claim 1, wherein the auxetic property of the support layer is provided by a plurality of openings (250) extending through the support layer and being spaced apart from each other.

3. The sensor patch according to claim 2, wherein each opening of the plurality of openings comprises at least a first opening portion (252) and a second opening portion (253) extending from a common start point (251) to a respective end point, the end point of the first opening portion being spaced from the end point of the second opening portion, wherein the common start point of a first opening (250A) of the plurality of openings is arranged on a first side of an imaginary straight line extending in a direction on the support layer and the common start point of a second opening (250B) of the plurality of openings is arranged on a second side of the straight line,
wherein the first opening portion of the first opening and the second opening portion of the second opening each cross the straight line.

4. The sensor patch according to any one of claims 2-3, wherein the plurality of openings forms a lattice pattern.

5. The sensor patch according to any one of claims 2-4, wherein at least one of the electrode paths extends between the first opening and the second opening.

6. The sensor patch according to any one of claims 2-5, wherein each opening portion of the plurality of openings is formed as a slit.

7. The sensor patch according to any one of claims 2-6, wherein an angle between one of the opening portions of an opening and another opening portion of the same opening is between 60° and 150°.

8. The sensor patch according to any one of claims 2-7, wherein each of the plurality of openings further comprises a third opening portion (254) extending from the common start point to a respective end point.

9. The sensor patch according to claim 8, wherein an angle between the second opening portion and the third opening portion of the same opening is between 60° and 120°.

10. A method for manufacturing a sensor patch (50) according to any one of claims 1-9, comprising
the steps of:
- providing a support layer (214) with a distal surface (214A) and a proximal surface (214B); and
- arranging a plurality of electrode paths (216) on the proximal surface of the support layer.

11. The method according to claim 10 for manufacturing a sensor patch according to any one of claims 2-9, wherein the support layer is provided with the plurality of openings pre-made.

12. The method according to claim 10 for manufacturing a sensor patch according to any one of claims 2-9, the method comprising a step of cutting the support layer to provide the plurality of openings in the support layer.

13. A method according to any one of claims 11-12, wherein the step of arranging the plurality of electrode paths on the proximal surface of the support layer comprises arranging the plurality of electrode paths to extend between the plurality of openings.

14. A kit of parts for an ostomy appliance, the kit of parts comprising:
- a sensor patch (50) according to any one of claims 1-9; and
- a concave or convex base plate comprising an adhesive surface (52, 56) adapted for attachment to a skin surface of a user, the base plate forming a stomal opening (18) with a centre point, the stomal opening being configured to allow passage of output from the user through the stomal opening and into an ostomy pouch attached to the base plate.

15. The kit of parts according to claim 14, wherein the distal surface of the sensor patch is attached to the convex or concave adhesive surface of the base plate substantially without creases.

## Patentansprüche

1. Sensorpflaster (50) zum Anbringen an einer konkaven oder konvexen Basisplatte für eine Stomaversorgung, wobei das Sensorpflaster eine proximale Seite und eine distale Seite aufweist, wobei die distale Seite zum Anbringen an einer Klebefläche der Basisplatte eingerichtet ist, wobei die Klebefläche der Basisplatte zum Anbringen der Basisplatte an der Hautoberfläche eines Benutzers eingerichtet ist, wobei das Sensorpflaster dazu eingerichtet ist, eine Stomaöffnung (18) mit einem Mittelpunkt zu bilden, wobei die Stomaöffnung dazu ausgelegt ist, den Durchfluss von Ausscheidungen durch die Stomaöffnung und in einen an der Grundplatte befestigten Stomabeutel zuzulassen, wobei das Sensorpflaster umfasst:
- eine Trägerschicht (214) mit einer distalen Oberfläche (214A) und einer proximalen Oberfläche (214B);
- eine Vielzahl von Elektrodenbahnen (216) zum Bilden eines oder mehrerer Sensoren, wobei die Vielzahl von Elektrodenbahnen auf der proximalen Oberfläche der Trägerschicht angeordnet ist; und
- eine Klebeschicht (52, 56), die auf der proximalen Oberfläche der Trägerschicht angeordnet ist;
**dadurch gekennzeichnet, dass**
die Trägerschicht auxetisch ist, um zu ermöglichen, dass sich das Sensorpflaster an die konkave oder konvexe Grundplatte anpasst.

2. Sensorpflaster gemäß Anspruch 1, wobei die auxetische Eigenschaft der Trägerschicht durch eine Vielzahl von Öffnungen (250) bereitgestellt wird, die sich durch die Trägerschicht erstrecken und voneinander beabstandet sind.

3. Sensorpflaster gemäß Anspruch 2, wobei jede Öffnung der Vielzahl von Öffnungen mindestens einen ersten Öffnungsabschnitt (252) und einen zweiten Öffnungsabschnitt (253) umfasst, die sich von einem gemeinsamen Anfangspunkt (251) zu einem jeweiligen Endpunkt erstrecken, wobei der Endpunkt des ersten Öffnungsabschnitts vom Endpunkt des zweiten Öffnungsabschnitts beabstandet ist, wobei der gemeinsame Anfangspunkt einer ersten Öffnung (250A) der Vielzahl von Öffnungen auf einer ersten Seite einer imaginären Geraden angeordnet ist, die sich in einer Richtung auf der Trägerschicht erstreckt, und der gemeinsame Anfangspunkt einer zweiten Öffnung (250B) der Vielzahl von Öffnungen auf einer zweiten Seite der Geraden angeordnet ist, wobei der erste Öffnungsabschnitt der ersten Öffnung und der zweite Öffnungsabschnitt der zweiten Öffnung jeweils die Gerade kreuzen.

4. Sensorpflaster gemäß einem der Ansprüche 2-3, wobei die Vielzahl von Öffnungen ein Gittermuster bildet.

5. Sensorpflaster gemäß einem der Ansprüche 2-4, wobei sich mindestens eine der Elektrodenbahnen zwischen der ersten Öffnung und der zweiten Öffnung erstreckt.

6. Sensorpflaster gemäß einem der Ansprüche 2-5, wobei jeder Öffnungsabschnitt der Vielzahl von Öffnungen als Schlitz ausgebildet ist.

7. Sensorpflaster gemäß einem der Ansprüche 2-6, wobei ein Winkel zwischen einem der Öffnungsabschnitte einer Öffnung und einem anderen Öffnungsabschnitt derselben Öffnung zwischen 60° und 150° liegt.

8. Sensorpflaster gemäß einem der Ansprüche 2-7, wobei jede der Vielzahl von Öffnungen ferner einen dritten Öffnungsabschnitt (254) umfasst, der sich von dem gemeinsamen Anfangspunkt zu einem jeweiligen Endpunkt erstreckt.

9. Sensorpflaster gemäß Anspruch 8, wobei der Winkel zwischen dem zweiten Öffnungsabschnitt und dem dritten Öffnungsabschnitt derselben Öffnung zwischen 60° und 120° liegt.

10. Verfahren zum Herstellen eines Sensorpflasters (50) gemäß einem der Ansprüche 1 bis 9, die Schritte umfassend:
- Bereitstellen einer Trägerschicht (214) mit einer distalen Oberfläche (214A) und einer proximalen Oberfläche (214B); und
- Anordnen einer Vielzahl von Elektrodenbahnen (216) auf der proximalen Oberfläche der Trägerschicht.

11. Verfahren gemäß Anspruch 10 zum Herstellen eines Sensorpflasters gemäß einem der Ansprüche 2-9, wobei die Trägerschicht mit einer Vielzahl von vorgefertigten Öffnungen versehen ist.

12. Verfahren gemäß Anspruch 10 zum Herstellen eines Sensorpflasters gemäß einem der Ansprüche 2-9, wobei das Verfahren einen Schritt des Schneidens der Trägerschicht umfasst, um die Vielzahl von Öffnungen in der Trägerschicht bereitzustellen.

13. Verfahren gemäß einem der Ansprüche 11-12, wobei der Schritt des Anordnens der Vielzahl von Elektrodenbahnen auf der proximalen Oberfläche der Trägerschicht umfasst, die Vielzahl von Elektrodenbahnen derart anzuordnen, dass sie sich zwischen der Vielzahl von Öffnungen erstrecken.

14. Teilesatz für eine Stomaversorgung, wobei der Teilesatz umfasst:
- ein Sensorpflaster (50) gemäß einem der Ansprüche 1-9; und
- eine konkave oder konvexe Basisplatte mit einer Klebefläche (52, 56), die zur Befestigung an einer Hautoberfläche eines Benutzers eingerichtet ist, wobei die Basisplatte eine Stomaöffnung (18) mit einem Mittelpunkt bildet, wobei die Stomaöffnung dazu ausgelegt ist, den Durchfluss von Ausscheidungen durch die Stomaöffnung und in einen an der Grundplatte befestigten Stomabeutel zuzulassen.

15. Teilesatz gemäß Anspruch 14, wobei die distale Oberfläche des Sensorpflasters im Wesentlichen faltenfrei an der konvexen oder konkaven Klebefläche der Grundplatte befestigt ist.

## Revendications

1. Patch capteur (50) destiné à être fixée à une plaque de base concave ou convexe pour un appareil de stomie, le patch capteur ayant un côté proximal et un côté distal, le côté distal étant adapté pour être fixé à une surface adhésive de la plaque de base, la surface adhésive de la plaque de base étant adaptée pour la fixation de la plaque de base à la surface de la peau d'un utilisateur, le patch capteur étant adapté pour former une ouverture stomale (18) avec un point central, l'ouverture stomale étant configurée pour permettre le passage d'excreta à travers l'ouverture stomale et jusqu'à l'intérieur d'une poche de stomie fixée à la plaque de base, le patch capteur comprenant :
- une couche de support (214) avec une surface distale (214A) et une surface proximale (214B) ;
- une pluralité de chemins d'électrode (216) pour former un ou plusieurs capteurs, la pluralité de chemins d'électrode étant agencée sur la surface proximale de la couche de support ; et
- une couche adhésive (52, 56) agencée sur la surface proximale de la couche de support ;
**caractérisé en ce que**
la couche de support est auxétique de manière à permettre la conformation du patch capteur avec la plaque de base concave ou convexe.

2. Patch capteur selon la revendication 1, la propriété auxétique de la couche de support étant fournie par une pluralité d'ouvertures (250) s'étendant à travers la couche de support et étant espacées les unes des autres.

3. Patch capteur selon la revendication 2, chaque ouverture de la pluralité d'ouvertures comprenant au moins une première partie d'ouverture (252) et une deuxième partie d'ouverture (253) s'étendant d'un point de départ commun (251) à un point d'extrémité respectif, le point d'extrémité de la première partie d'ouverture étant espacé du point d'extrémité de la deuxième partie d'ouverture, le point de départ commun d'une première ouverture (250A) de la pluralité d'ouvertures étant agencé sur un premier côté d'une ligne droite imaginaire s'étendant dans une direction sur la couche de support et le point de départ commun d'une seconde ouverture (250B) de la pluralité d'ouvertures étant agencé sur un second côté de la ligne droite, la première partie d'ouverture de la première ouverture et la deuxième partie d'ouverture de la seconde ouverture croisant chacune la ligne droite.

4. Patch capteur selon l'une quelconque des revendications 2 et 3, la pluralité d'ouvertures forme un motif en treillis.

5. Patch capteur selon l'une quelconque des revendications 2 à 4, au moins l'un des chemins d'électrode s'étendant entre la première ouverture et la seconde ouverture.

6. Patch capteur selon l'une quelconque des revendications 2 à 5, chaque partie d'ouverture de la pluralité d'ouvertures étant formée comme une fente.

7. Patch capteur selon l'une quelconque des revendications 2 à 6, un angle entre l'une des parties d'ouverture d'une ouverture et une autre partie d'ouverture de la même ouverture étant compris entre 60° et 150°.

8. Patch capteur selon l'une quelconque des revendications 2 à 7, chacune de la pluralité d'ouvertures comprenant en outre une troisième partie d'ouverture (254) s'étendant du point de départ commun à un point d'extrémité respectif.

9. Patch capteur selon la revendication 8, un angle entre la deuxième partie d'ouverture et la troisième partie d'ouverture de la même ouverture étant compris entre 60° et 120°.

10. Procédé de fabrication d'un patch capteur (50) selon l'une quelconque des revendications 1 à 9, comprenant les étapes de :
- fourniture d'une couche de support (214) avec une surface distale (214A) et une surface proximale (214B) ; et
- agencement d'une pluralité de chemins d'électrodes (216) sur la surface proximale de la couche de support.

11. Procédé selon la revendication 10 pour fabriquer un patch capteur selon l'une quelconque des revendications 2 à 9, la couche de support étant pourvue de la pluralité d'ouvertures préfabriquées.

12. Procédé selon la revendication 10 pour fabriquer un patch capteur selon l'une quelconque des revendications 2 à 9, le procédé comprenant une étape de coupe de la couche de support pour fournir la pluralité d'ouvertures dans la couche de support.

13. Procédé selon l'une quelconque des revendications 11 à 12, l'étape d'agencement de la pluralité de chemins d'électrode sur la surface proximale de la couche de support comprenant l'agencement de la pluralité de chemins d'électrode pour s'étendre entre la pluralité d'ouvertures.

14. Kit de pièces pour un appareil de stomie, le kit de pièces comprenant :
- un patch capteur (50) selon l'une quelconque des revendications 1 à 9 ; et
- une plaque de base concave ou convexe comprenant une surface adhésive (52, 56) adaptée pour être fixée à la surface de la peau d'un utilisateur, la plaque de base formant une ouverture stomale (18) avec un point central, l'ouverture stomale étant configurée pour permettre le passage d'excreta de l'utilisateur à travers l'ouverture stomale et dans une poche de stomie fixée à la plaque de base.

15. Kit de pièces selon la revendication 14, la surface distale du patch capteur étant fixée à la surface adhésive convexe ou concave de la plaque de base sensiblement sans plis.
